# EUROPÄISCHE PATENTSCHRIFT

(11) **EP 0 992 481 B1**
(45) Veröffentlichungstag und Bekanntmachung des Hinweises auf die Patenterteilung: **21.05.2003**
(21) Anmeldenummer: 99118428.4
(22) Anmeldetag: 17.09.1999
(51) Int. Cl.: C07C 67/31, C07C 69/675

(54) **Verfahren zur asymmetrische Hydrierung von Beta-Ketoestern**
Process for the asymmetric hydrogenation of beta-ketoesters
Procédé d'hydrogénation asymmétrique de béta-cétoesters

(30) Priorität: 02.10.1998 DE 19845517
(43) Veröffentlichungstag der Anmeldung: 12.04.2000
(73) Patentinhaber: BASF AKTIENGESELLSCHAFT, 67056 Ludwigshafen (DE)
(72) Erfinder: Stürmer, Rainer, Dr., 67127 Rödersheim-Gronau (DE); Klatt, Martin Jochen, Dr., 67098 Bad Dürkheim (DE); Börmer, Armin, Dr., 18050 Rostock (DE); Holz, Jens, Dr., 18057 Rostock (DE); Voss, Gudrun, 18055 Rostock (DE)

(56) Entgegenhaltungen:
- EP-A- 0 863 125
- EP-A- 0 889 048
- WO-A-92/19630
- ERNEST L. ELIEL: "Stereochemistry of Organic Compounds", 1994, WILEY & SONS, NEW YORK

## Beschreibung

Die vorliegende Erfindung betrifft ein Verfahren zur Herstellung von enantiomerenreinen β-Hydroxyestern durch Hydrierung in Gegenwart von Ruthenium-Katalysatoren.

Bekannt ist die katalytische Hydrierung von Ketonen und β-Ketoestern mit Ru-Diphosphin-komplexen (z.B. Burk et.al, J.Am.Chem.Soc 1995, 117, 4423; A. Mortreux et.al.Tetrahedron: Asymmetry, 7(2), 379-82, 1996; Noyori et.al. Angew. Chem., Int. Ed. Engl., 36(3), 285-288 , 1997; WO 9713763 A1).

EP-A-0 863 125 beschreibt die asymmetrische katalytische Hydrierung von 3-Oxo-Octansäurediestern zu 3-Hydroxyoctansäurediestern mit Ruthenium-Diphosphin und Diphospholan Katalysatoren.

Bekannt ist ebenfalls die katalytische Transfer-Hydrierung von Ketonen mit Ameisensäure / Triethylamin-Komplex als Reduktionsmittel und Rutheniumkatalysatoren (P. Knochel et.al. Tetrahedron Lett., 37(45), 8165-8168 , 1996; Sammakia et.al J. Org. Chem., 62(18), 6104-6105 , 1997 (Isopropanol als Reduktionsmittel).

Gemeinsam ist allen diesen Methoden, daß sehr aufwendig herzustellende Katalysatoren und Liganden verwendet werden. Bei den Transferhydrierungen wird ferner nicht der billige Wasserstoff, sondern Isopropanol oder Ameisensäure/Tert. Amine verwendet. Letzteres erschwert die Aufarbeitung der Reaktion und führt zum Zwangsanfall von Aceton bzw. Kohlendioxid.

Ferner wird bei diesen Reaktionen i.d. Regel mit sehr grossen Katalysatormengen gearbeitet; dies macht die bisherigen Verfahren unwirtschaftlich.

Aufgabe war es daher, ein Verfahren zur Hydrierung von Ketoestern zu finden, das mit Wasserstoff als Reduktionsmittel arbeitet, einen einfach herzustellenden Katalysator benutzt, ein hohes Substrat-Katalysator-Verhältnis erlaubt und welches mit hoher Enantioselektivität arbeitet.

Gegenstand der Erfindung ist ein Verfahren zur Herstellung von enantiomerenreinen β-Hydroxyestern, indem man β-Ketoester mit Wasserstoff in Gegenwart von Katalysatoren der Formel LRuX₂ umsetzt, wobei
- X: Halogen, Acetat, Allyl, Methallyl, 2-Phenylallyl, Perchlorat, Trifluoracetat, Tetrafluoroborat, Hexafluorantimonat, Hexafluorphosphat, Hexafluorarsenat, Trichloracetat,
- L: ein zweizähniges Phospholan der allgemeinen Formel I 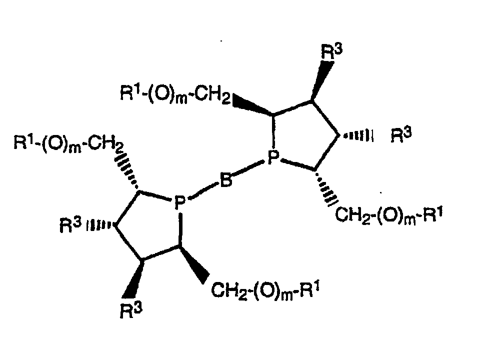
mit
B = ein Brückenglied mit 1 - 5 C-Atomen zwischen den beiden P-Atomen,
R¹ = H, C₁ - C₆-Alkyl, Aryl, Alkylaryl, SiR²₃
R² = Alkyl, Aryl
m = 0,1
R³ = H, OR⁴
R⁴ = R¹,
mit der Maßgabe, wenn m = 1, dann R³ = H und wenn m = 0, dann R³ ≠ H, bedeuten.

Bevorzugte Brückenglieder B sind solche, bei denen 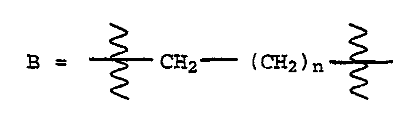 mit n = 0, 1, 2, 3, 4 oder 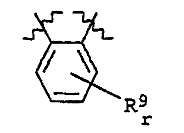 mit
r = 0, 1, 2, 3
R⁹ = Alkyl oder
   annelliertes Aryl

Besonders bevorzugt sind solche Brückenglieder B bei denen n=1 oder 2 oder r=0 ist.

Die Herstellung der zweizähnigen Phospholanliganden L ist in den Patentanmeldungen DE 19725796.8 und DE 19824121.6 und im experimentellen Teil dieser Anmeldung beschrieben.

Die Herstellung erfolgt ausgehend von dem Zucker Mannit, der aus natürlichen Quellen enantiomerenrein verfügbar ist.

Die Rutheniumkomplexe LRuX₂ lassen sich herstellen, indem man in bekannter Weise (z.B. Uson, Inorg. Chim. Acta 73, 275 (1983, EP-A 0158875, EP-A 437690) durch Umsetzung mit Rutheniumkomplexen, die labile Liganden enthalten (z.B. [RuCl₂(COD)]ₙ, p-Cymol-Rutheniumchlorid-dimer) katalytisch aktive Komplexe synthetisiert.

Die erfindungsgemäße Hydrierung wird in der Regel bei einer Temperatur von -20 bis 150°C, bevorzugt bei 0 bis 100°C und besonders bevorzugt bei 15 bis 40°C durchgeführt.

Der Wasserstoffdruck kann in einem großen Bereich zwischen 0 bis 300 bar, bzw. 0,1 bar und 300 bar für das erfindungsgemäße Hydrierverfahren variiert werden. Sehr gute Ergebnisse erhält man in einem Druckbereich von 1 bis 100, bevorzugt 1 bis 50 bar.

Die Umsetzung wird bevorzugt in einem Lösungsmittel, das ein Alkanol enthält, durchgeführt.

Bevorzugte Lösungsmittel für die Hydrierungen sind C₁-C₄-Alkanole, insbesondere MeOH. Bei schlecht löslichen Substraten sind auch Lösungsmittelgemische, z.B. Methanol und CH₂Cl₂, THF, Toluol oder auch Wasser geeignet.

Besonders bevorzugt wird dasjenige Alkanol verwendet, aus dem der umzusetzende β-Ketoester aufgebaut ist, da dadurch unerwünschte Umesterungen vermieden werden.

Der Katalysator wird üblicherweise in Mengen von 1:10 bis 1:1000000, bevorzugt 1:1000 bis 1:100 000 (w/w) bezogen auf das zu hydrierende Substrat, eingesetzt.

Die Umsetzung kann durch Zusatz einer Säure, besonders einer starken Säure wie Mineralsäuren oder Trifluor- oder Trichloressigsäuren sowohl hinsichtlich Ausbeute als auch hinsichtlich Selektivität verbessert werden.

Dazu wird im allgemeinen die Säure in einer Menge von 0,5 - 2 Mol Äquivalenten, bezogen auf Katalysator, zugesetzt.

### Experimenteller Teil

### Beispiel 1

### Herstellung eines Diphospholans L:

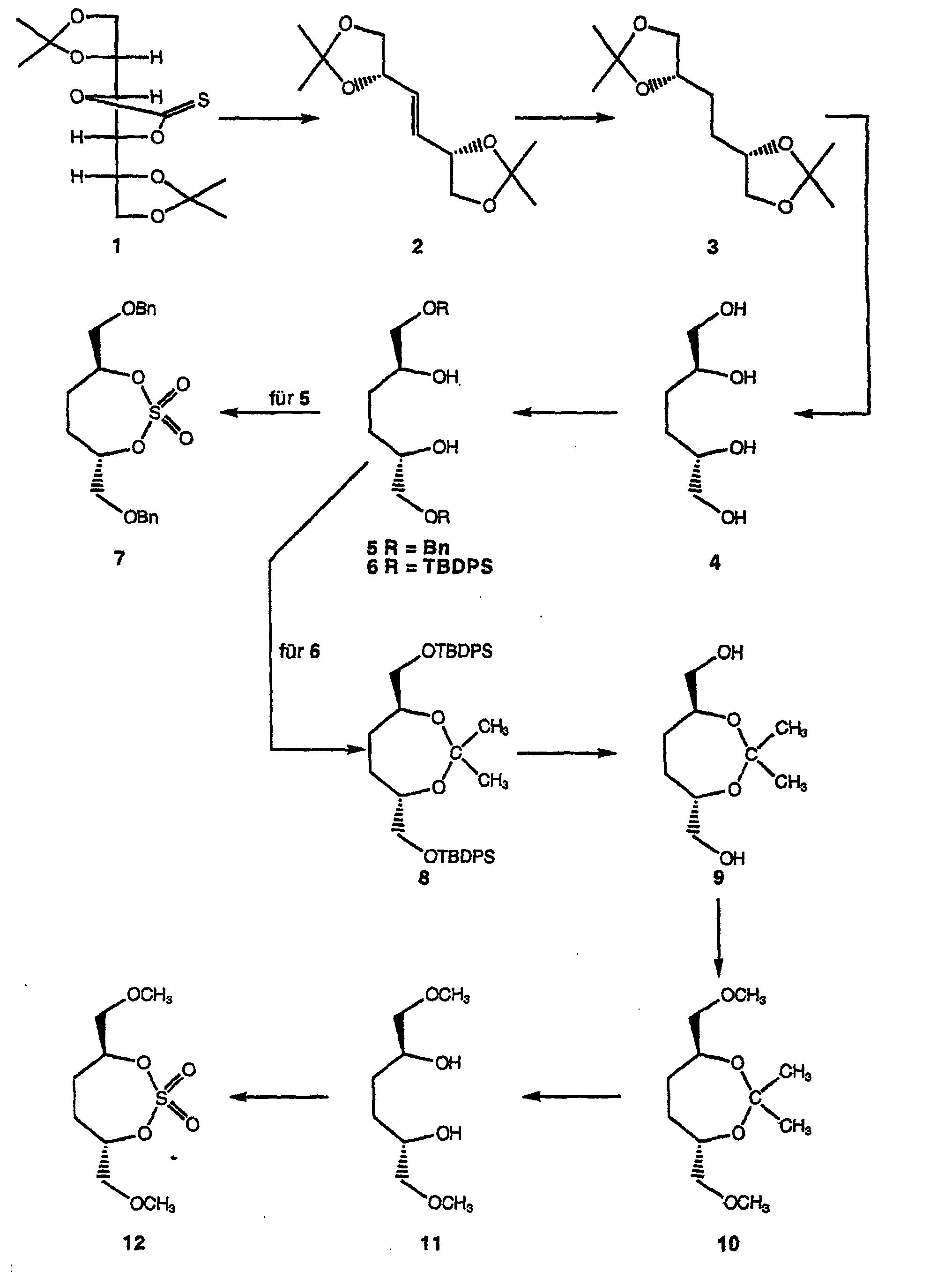

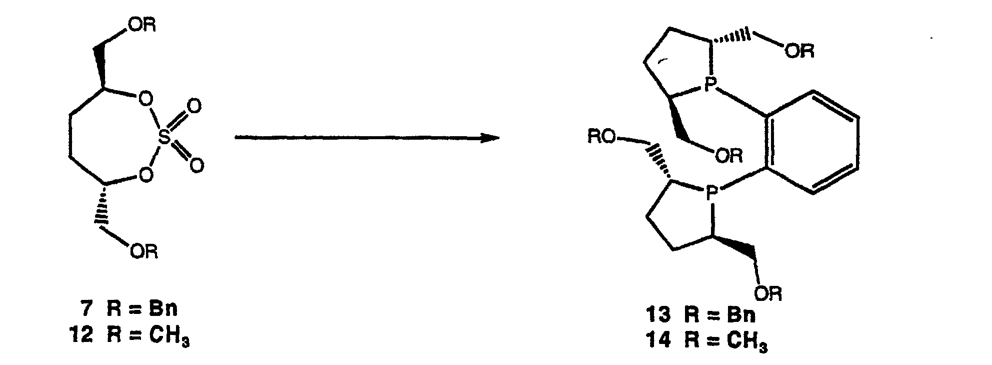

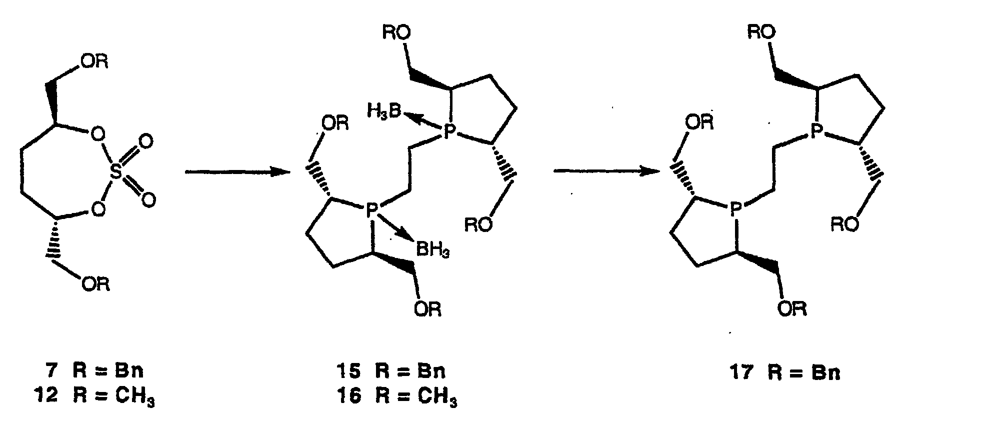

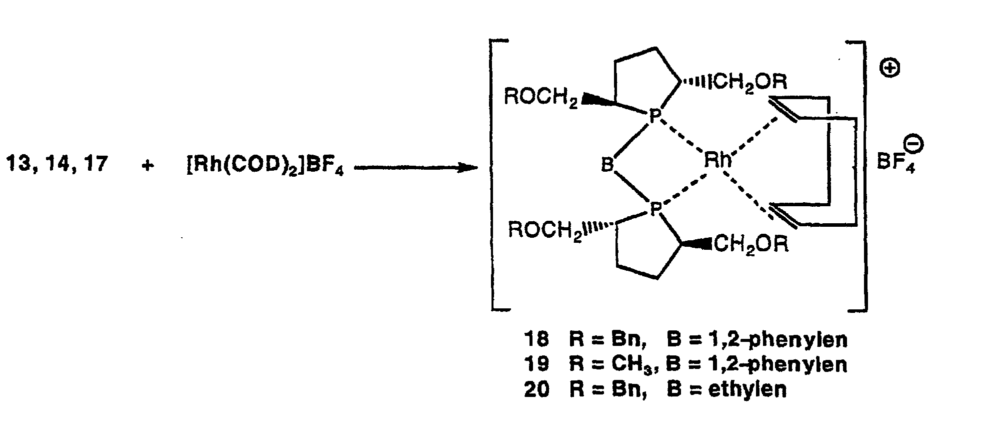

### 1,2;5,6-Di-O-isopropyliden-3,4-O-thiocarbonyl-D-mannitol (1):

Nach der Methode von E.J. Corey et al.¹ wurde das 1,2;5,6-Di-*O*isopropyliden-D-mannitol mit Thiophosgen in Gegenwart von 4-Dimethylaminopyridin in Methylenchlorid mit 90 %iger Ausbeute umgesetzt.

### E-3,4-Didehydro-3,4-didesoxy-1,2;5,6-di-O-isopropyliden-D-threohexitol (2):

Durch 20-stündiges Erhitzen des cyclischen Thiocarbonats 1 in Triethylphosphit nach der Literatur^{2,3} konnte das *trans*-Olefin in Ausbeuten von 80 bis 90 % gewonnen werden.

### 3,4-Didesoxy-1,2;5,6-di-O-isopropyliden-D-threo-hexitol (3):

In Abwandlung zur Methode von Machinaga et al.⁴ wurde das Olefin 2 (10 g) in Methanol mit 10 % Platin auf Aktivkohle (250 mg) bei Normaldruck zur Verbindung **3** hydriert. Nach der säulenchromatographischen Reinigung betrug die Ausbeute 80 bis 90 %. Eine destillative Reinigung von Verbindung **3** nach der Literatur⁴ ist ebenfalls möglich (Sdp. 0,6 [mm Hg] 79,8 Pa = 73°C).

### 3,4-Didesoxy-D-threo-hexitol (4):

Die saure Hydrolyse der Isopropylidengruppen erfolgte entsprechend der Literatur⁴ in 1N Salzsäure. Die Verbindung wurde nach dem Umkristallisieren in einer Ausbeute von 85 % erhalten.

### (2S,5S)-1,6-Bis(benzyloxy)-hexan-2,5-diol (5):

Entsprechend der Vorschrift von Marzi et al.⁵ wurden 3,0 g (20 mmol) des 3,4-Didesoxy-D-*threo*-hexitols (4) in 3,70 g des 1,6-di-*O*-benzylierten Produktes 5 in einer Ausbeute von 56 % überführt.

### (2S,5S)-1,6-Bis[(tert.-butyldiphenylsilyl)oxy)-hexan-2,5-diol (6):

In Anlehnung an die Literatur⁵ wurden 3,0 g (20 mmol) der Verbindung 4 in DMF mit *tert.*-Butyldiphenylchlorsilan in Gegenwart von Imidazol zum Derivat 6 in einer Ausbeute von 80 % umgesetzt.

### (4S,7S)-4,7-Bis(benzyloxymethyl)-2,2-dioxo-[1,3,2]-dioxothiepan (7):

3,30 g (10 mmol) des Diols 5 wurden in 70 ml trockenem Tetrachlorkohlenstoff unter Argonatmosphäre mit 1,43 g (12 mmol) Thionylchlorid langsam versetzt und anschließend 90 Minuten unter Rückfluß erhitzt. Nach dem Entfernen des Lösungsmittels am Rotationsverdampfer wurde der Rückstand mit einer Mischung von Tetrachlorkohlenstoff (40 ml), Acetonitril (40 ml) und Wasser (60 ml) aufgenommen und bei 0°C mit 15 mg (72 µmol) RuCl₃*3H₂O und 4,28 g (20 mmol) Natriumperiodat versetzt. Man ließ dann eine Stunde bei Raumtemperatur rühren und versetzte dann die Suspension mit 50 ml Wasser. Durch anschließende Extraktion mit Diethylether (3 x 75 ml) und Waschen der organischen Phase mit gesättigter NaCl-Lösung (100 ml) erhielt man nach dem Trocknen (Na₂SO₄) einen Rückstand, welcher säulenchromatographisch (n-Hexan:AcOEt = 2:1, R_{f} = 0.20) die Verbindung 7 in einer Ausbeute von 3.37 g (86 %) lieferte.
Smp. = 57 bis 59°C; [α]_{D}²⁶ = -37,2° (c 1.01; CHCl₃); ¹H-NMR (CDCl₃, 400 MHz) δ 7,34 (10H, m, arom. H), 4,78 (2H, m, H-2/5), 4,57 (2H, AB-Sp., Hₐ-CH₂Ph, ²J_{a,b}= 12,0 Hz), 4,56 (2H, AB-Sp., H_{b}-CH₂Ph, ²J_{a,b} = 12.0 Hz), 3,65 (2H, dd, Hₐ-CH₂OH, ²J_{a,b} = 10,8 Hz, ³J_{H,H} = 5,4 Hz), 3,56 (2H, dd, H_{b}-CH₂OH, ²J_{a,b} = 10,8 Hz, ³J_{H,H} = 4.9 Hz), 2,00 (4H, m, H-3/4); ¹³C-NMR (CDCl₃, 100 MHz) δ 137,3, 128,4-127.7 (arom. C), 82,6 (C-2/5), 73,4 (CH₂Ph), 70,8 (C-1/6), 28,9 (C-3/4); Elementaranalyse C₂₀H₂₄O₆S (392,47) ber.: C 61,21, H 6,16, S 8,17; gef. C 61,03, H 6,19, S 8,10;

### 1,6-Di-O-(tert.-butyldiphenyl)silyl-2,5-di-O-isopropyliden-3,4-didesoxy-D-threo-hexitol (8):

Entsprechend der Literatur⁵ wurden 6,27 g (10 mmol) der Verbindung 6 in einer Ausbeute von 85 % (5,67 g) zum Isopropyliden-Derivat 8 umgesetzt. Die Reinigung von 8 zur Charakterisierung erfolgte durch Säulenchromatographie (*n*-Hexan: Diethylether = 19:1, R_{f} = 0.2). Für den nächsten Reaktionsschritt konnte auf eine Reinigung der Verbindung verzichtet werden.

### 2,5-Di-O-isopropyliden-3,4-didesoxy-D-threo-hexitol (9):

Aus 6,67 g (10 mmol) der Silylverbindung 8 wurden nach der Abspaltung der Silylgruppen mit Tetrabutylammoniumfluorid in THF⁵ und der anschließenden chromatographierte Reinigung (Diethylether:MeOH = 19:1, R_{f} = 0,5) 1,7 g (89 %) des Diols 9 erhalten.

### 2, 5-Di-O-isopropyliden-1,6-di-O-methyl-3,4-didesoxy-D-threohexitol (10):

Eine Lösung von 3,80 g (20 mmol) des Diols 9 in 30 ml THF wurden bei 0°C zu einer Lösung von 1,06 g (44 mmol) NaH in 60 ml THF gegeben. Nach Beendigung der Alkoholatbildung addierte man langsam 2,2 Äquivalente Methyliodid (6,21 g, 44 mmol) hinzu und ließ bei Raumtemperatur rühren. Nach Abschluß der Reaktion vernichtete man den Überschuß von NaH vorsichtig mit Wasser (30 ml) und entfernte unter Vakuum das THF. Die verbleibende wäßrige Lösung wurde dann mit Methylenchlorid (3 x 50 ml) extrahiert und die vereinigte organischen Phase getrocknet (Na₂SO₄). Der nach dem Einengen erhaltene Rückstand lieferte nach der Säulenchromatographie (*n*-Hexan:AcOEt = 2:1, R_{f} = 0.40) einen farblosen Sirup in einer Ausbeute von 84 % (3,68 g).
Sirup; [α]_{D}²³ = -32,8° (*c* 1,01, CHCl₃); ¹H-NMR (CDCl₃, 400 MHz) δ 3,92 (2H, m, H-2/5), 3,32 (2H, dd, Hₐ-CH₂O, ²J_{a,b} = 9,9 Hz, ³J_{H,H} = 6,3 Hz), 3,30 (6H, s, CH₃), 3,55 (2H, m, H_{b}-CH₂O, ²J_{a,b} = 9,9 Hz, ³J_{H,H} = 5,3 Hz), 1,67 (2H, m, Hₐ-3/4), 1,34 (2H, m, H_{b}-3/4), 1,31 (6H, s, CH₃); ¹³C-NMR (CDCl₃, 100 MHz) δ 100,5 (C(O)₂), 76,2 (C-1/6), 70,4 (C-2/5), 59,1 (CH₃), 31,1 (C-3/4), 25,6 (C(CH₃)₂); Elementaranalyse C₁₁H₂₂O₄ (218.293) ber.: C 60,52, H 10,16; gef.: C 60,38, H 10,07;

### (2S,5S)-1,6-Bis(benzyloxy)-hexan-2,5-diol (11):

4,0 g (18,32 mmol) der Verbindung 10 wurden in einer Mischung von 60 ml THF und 60 ml 1N Salzsäure innerhalb von 20 Minuten hydrolysiert. Nach dem Einengen der Lösung am Rotationsverdampfer wurden auf chromatographischem Weg (EtOH:AcOEt = 1:3, R_{f} = 0,45) in nahezu quantitativer Ausbeute 3,20 g eines schwach gelben Sirups 11 erhalten.
Sirup; [α]_{D}²² = -7,2° (c 1,09, CH₃OH); ¹H-NMR (CD₃OD, 400 MHz) δ 3,72 (2H, m, H-2/5), 3,37 (6H, s, CH₃), 3,38-3,30 (4H, m, CH₂OH), 1,56 (4H, m, H-3/4); ¹³C-NMR (CD₃OD, 100 MHz) δ 78,2 (C-1/6), 70,1 (C-2/5), 59,2 (CH₃), 30,6 (C-3/4); Elementaranalyse C₈H₁₈O₄ (178.228) ber.: C 53,91, H 10,18; gef.: C 53,47, H 10,14;

### (4S,7S)-4,7-Bis(methyloxymethyl)-2,2-dioxo-[1,3,2]-dioxothiepan (12):

In Analogie zur Darstellung des cyclischen Sulfats 7 wurden 1,78 g (10 mmol) des Diols 11 in die Zielverbindung 12 überführt. Auf eine chromatographische Reinigung (*n*-Hexan:AcOEt = 1:2, R_{f} = 0,4) konnte hier verzichtet werden, denn das Produkt 12 ließ sich durch Kristallisation aus Diethylether/*n*-Hexan in einer Ausbeute von 76 % (1,83 g) als weißer Feststoff isolieren. Smp. = 75-78°C; [α]_{D}²³= -44,1° (*c* 1,01; CHCl₃); ¹H-NMR (CDCl₃, 400 MHz) δ 4,72 (2H, m, H-2/5), 3,56 (2H, dd, Hₐ-CH₂O, ²J_{a,b}= 10,8 Hz, ³J_{H,H} = 5,4 Hz), 3,47 (2H, dd, Hₐ-CH₂O, ²J_{a,b} = 10,8 Hz, ³J_{H,H} = 4,7 Hz), 3,37 (6H, s, CH₃), 2,04-1,92 (4H, m, H-3/4); ¹³C-NMR (CDCl₃, 100 MHz) δ 82,5 (C-2/5), 73,4 (C-1/6), 59,3 (OCH₃), 28,8 (C-3/4); Elementaranalyse C₈H₁₆O₆S (240.274) ber.: C 39,99, H 6,71, S 13,34; gef.: C 40.06, H 6,76, S 1,.27;

### 1,2-Bis[(2R,5R)-2,5-benzyloxymethylphospholanyl]benzen (13):

0,52 g (3,66 mmol) 1,2-Bis(phosphanyl)benzen in 50 ml THF wurden mit 2,0 Äquivalenten *n*-BuLi (4,58 ml, 1,6 M Lösung in *n*-Hexan) langsam versetzt und nach zwei Stunden gab man zu der resultierenden gelben Lösung langsam 2,86 g (7,32 mmol) des cyclischen Sulfats 7 in 20 ml THF hinzu. Man ließ weitere 2 Stunden bei Raumtemperatur rühren und addierte schließlich erneut 2,2 Äquivalente *n*-BuLi (5,03 ml, 1,6 M Lösung in *n-*Hexan) hinzu. Die Lösung ließ man über Nacht rühren und vernichteten Überschuß an BuLi schließlich mit 2 ml MeOH. Das Lösungsmittel wurde unter Vakuum entfernt und der Rückstand unter anaeroben Bedingungen mit 20 ml Wasser aufgenommen und dann mit Methylenchlorid (2 x 50 ml) extrahiert. Nach dem Trocknen der organischen Phase (Na₂SO₄) und Entfernen des Solvens isolierte man das gewünschte Produkt durch Säulenchromatographie (n-Hexan:AcOEt = 4:1, R_{f} = 0,35) in einer Ausbeute von 0,52 g (19 %) als schwach-gelben Sirup.
Sirup; ¹H-NMR (CDCl₃, 400 MHz) δ 7,45-7,10 (24H, m, arom. H), 4,49 (2H, AB-Sp., Hₐ-CH₂Ph, ²J_{a,b} = 12.1 Hz), 4,47 (2H, AB-Sp., H_{b}-CH₂Ph, ²J_{a,b} = 12,1 Hz), 4,18 (2H, AB-Sp., Hₐ-CH₂Ph, ²J_{a,b} = 11,9 Hz), 4,04 (2H, AB-Sp., H_{b}-CH₂Ph, ²J_{a,b} = 11,9 Hz), 3,65-3,45 (4H, m, CH₂O), 2.97-2.80 (4H, m, CH₂O), 2.70 (2H, m, CH-P); 2.33 (4H, m, CH-P, Hₐ-(CH₂)₂); 2,18 (2H, m, Hₐ-(CH₂)₂), 1,80-1,53 (4H, m, H_{b}-(CH₂)₂); ¹³C-NMR (CDCl₃, 100 MHz) δ 141,8 (m, Cₐᵣ-P), 138,6+138,5 (*ipso-C*), 131,8, 128,4-127,1 (arom. C), 74,1 (m, CH₂Ph), 73,0 (CH₂Ph), 72,5 (CH₂O), 72,5 (CH₂O), 39,5 (CH-P), 38,9 (m, CH-P), 30,9 (CH₂), 30,4 (CH₂); ³¹P-NMR (CDCl₃, 162 MHz) δ-11,5;

### 1,2-Bis[(2R,5R)-2,5-benzyloxymethylphospholanyl]benzen (14):

Analog zur Darstellung von Bisphospholan 13 wurde an Stelle des cyclischen Sulfats 7 die Verbindung 12 zum gewünschten Methoxymethyl-substituierten Bisphospholan 14 umgesetzt. Die Reinigung und Isolierung erfolgte durch Säulenchromatographie (n-Hexan: AcOEt = 2:1, R_{f} = 0,20) in einer Ausbeute von 0,80 g (48 %) des farblosen Sirups.

Sirup; ¹H-NMR (CDCl₃, 400 MHz) δ 7,45 (2H, m, arom. H), 7,30 (2H, m, arom. H), 3,55 (4H, m, CH₂O), 3,36 (2H, m, CH₂O), 3,35 (6H, s, CH₃), 3,10 (6H, s, CH₃), 2,90 (2H, m, CH₂O), 2,78 (2H, m, CH-P), 2,63 (2H, m, CH-P), 2,32 (2H, m, CH₂); 2,16 (4H, m, CH₂); 1,68 (2H, m, CH₂), 1,55 (4H, m, CH₂); ¹³C-NMR (CDCl₃, 100 MHz) δ 141,9 (m, Cₐᵣ-P), 131,8, 128,4 (arom. C), 74,1 (m, CH₂Ph), 76,6 (m, CH₂O), 74,5 (CH₂O), 58,8 (CH₃), 58,2 (CH₃), 39,6 (CH-P), 39,0 (m, CH-P), 30,9 (CH₂), 30,3 (CH₂); ³¹P-NMR (CDCl₃, 162 MHz) δ -11,7;

### 1,2-Bis[(2R, 5R)-2,5-benzyloxymethylphospholanyl]ethan Borankomplex (15):

348 mg (3,70 mmol) Bis(phosphanyl)ethan wurden bei Raumtemperatur in THF mit 7,40 mmol (4,63 ml) einer 1,6 M *n*-BuLi-Lösung in Hexan versetzt und zwei Stunden gerührt. Dann gab man langsam eine Lösung von 2,90 g (7,40 mmol) des cyclischen Sulfats 7 in 20 ml THF hinzu und rührte weitere zwei Stunden. Durch anschließende Zugabe weiterer 5,09 ml (8,14 mmol) n-BuLi-Lösung wurde die Reaktion komplettiert und über Nacht gerührt. Zur Bildung des Boran-Adduktes kühlte man die Lösung auf -20°C ab und gab 9,25 ml (9,25 mmol) einer 1M BH₃*THF-Lösung hinzu. Nach zwei Stunden vernichtete man überschüssiges BuLi und BH₃ durch Zugabe von 2 ml MeOH und entfernte das Lösungsmittel unter Vakuum. Der Rückstand wurde mit Wasser aufgenommen und dann mit Methylenchlorid extrahiert. Die Extrakte wurden dann getrocknet (Na₂SO₄) und eingeengt und der verbleibende Rückstand säulenchromatographisch (*n*-Hexan: AcOEt = 4:1, R_{f} = 0,20) gereinigt. Man erhielt 350 mg (13 %) eines zähflüssigen Sirups.

Sirup; ¹H-NMR (CDCl₃, 400 MHz) δ 7,37-7,22 (20H, m, arom. H), 4,47 (2H, AB-Sp., Hₐ-CH₂Ph, ²J_{a,b} = 11,2 Hz), 4,42 (2H, AB-Sp., Hₐ-CH₂Ph, ²J_{a,b} = 12,1 Hz), 4,41 (2H, AB-Sp., H_{b}-CH₂Ph, ²J_{a,b} = 12,1 Hz), 4, 38 (2H, AB-Sp., H_{b}-CH₂Ph, ²J_{a,b} = 11,2 Hz), 3,58 (4H, m, CH₂O), 3,43 (4H, m, CH₂O), 2,37 (2H, m, CH-P); 2,14-1,79 (10H, m, CH-P, (CH₂)₂), 1,41-1,20 (2H, m, (CH₂)₂), 0,85-0,00 (6H, m, BH₃); ¹³C-NMR (CDCl₃, 100 MHz) δ 138,1+137,9 (*ipso*-C), 128,3-127,4 (arom. C), 73,2 (CH₂Ph), 72,7 (CH₂Ph), 69,4 (CH₂O), 68,4 (CH₂O), 39,5 (m, CH-P), 29,1 (CH₂), 28,6 (CH₂), 15,9 (m, CH₂)₂); ³¹P-NMR (CDCl₃, 162 MHz) δ 40,2;

### 1,2-Bis[(2R,5R)-2,5-methyloxymethylphospholanyl]ethan Borankomplex (16):

In Analogie zur Darstellung der Verbindung 15 wurden 2,14 g (8,91 mmol) cyclisches Sulfat 12 und 0,42 g (4,45 mmol) Bis(phosphanyl)ethan zum gewünschten borangeschützten Bisphospholan 16 umgesetzt. Die chromatographische Reinigung erfolgte mit *n*-Hexan:AcOEt = 2:1 (R_{f} = 0,15). Man erhielt ein kristallines Produkt in einer Ausbeute von 0,71 g (39 %).

Smp.= 45-48°C; [α]_{D}²³ = 21,9° (*c* 1.00; CHCl₃); ¹H-NMR (CDCl₃, 400 MHz) δ 3,51 (8H, m, CH₂O), 3,33 (6H, s, CH₃O), 3,32 (6H, m, CH₃O), 2,36 (2H, m, CH-P); 2,23-2,05 (6H, m, CH-P, (CH₂)₂), 1,96 (4H, m, CH₂)₂), 1,58-1,35 (4H, m, (CH₂)₂), 0,95-0,00 (6H, m, BH₃); ¹³C-NMR (CDCl₃, 100 MHz) δ 71,6 (m, CH₂O), 70,8 (CH₂O), 58,7 (CH₃O), 58,7 (CH₃O), 39,5 (m, CH-P), 29,1 (CH₂), 28,9 (CH₂), 15,8 (m, CH₂)₂); ³¹P-NMR (CDCl₃, 162 MHz): δ 40,5; MS (m/z; EI) 391 [M⁺-BH₄] (100);

### 1,2-Bis[(2R,5R)-2,5-benzyloxymethylphospholanyl]ethan (17):

0,30 g (0,42 mmol) des Boran-Komplex 15 wurden mit einer aneoroben Lösung von 0,142 g (1,26 mmol) DABCO in 6 ml Toluol versetzt und bei 40°C gerührt. Nach Vollständigkeit der Reaktion wurde die Lösung eingeengt und rasch säulenchromatographisch gereinigt (*n*-Hexan:AcOEt = 4:1, R_{f}= 0.55). Das Bisphospholan 17 wurde in einer Ausbeute von 0,21 g (73 %) erhalten und sofort zur Komplexbildung eingesetzt.

Sirup; ¹H-NMR (CDCl₃, 400 MHz) δ 7,35-7,21 (20H, m, arom. H), 4,52 (2H, AB-Sp., Hₐ-CH₂Ph, ²J_{a,b} = 12,1 Hz), 4,48 (2H, AB-Sp., H_{b}-CH₂Ph, ²J_{a,b} = 12,1 Hz), 4,43 (2H, AB-Sp., Hₐ-CH₂Ph, ²J_{a,b} = 12,1 Hz), 4,41 (2H, AB-Sp., H_{b}-CH₂Ph, ²J_{a,b} = 12,1 Hz), 3,61-3,41 (8H, m, CH₂O), 2,29 (2H, m, CH-P); 2,20 (2H, m, CH-P); 2,07 (4H, m, Hₐ-(CH₂)₂), 1,53-1,23 (8H, m, H_{b}-(CH₂)₂), (CH₂)₂); ¹³C-NMR (CDCl₃, 100 MHz) δ 138,6+138,4 (*ipso*-C), 128,3-127,3 (arom. C), 74,2 (m, CH₂Ph), 72,9 (CH₂Ph), 72,7 (CH₂O), 70,2 (CH₂O), 43,7 (m, CH-P), 40,0 (m, CH-P), 31,4 (CH₂), 31,3 (CH₂), 19,1 (m, CH₂)₂); ³¹P-NMR (CDCl₃, 162 MHz): δ -6,9;

### Literatur

¹ E.J. Corey; P.B. Hopkins *Tetrahedron Lett.* 23 (1982) 1979-1982;
² M. Marzi; D. Misiti *Tetrahedron Lett.* 30 (1989) 6075-6076;
³ A. Haines *Carbohydrate Res.* 1 (1965) 214-228;
⁴ N. Machinaga; C. Kibayashi *J. Org. Chem.* 57 (1992) 5178-5189;
⁵ M. Marzi; P. Minetti; D. Misiti *Tetrahedron* 48 (1992) 10127-10132;

### Beispiel 2

### Darstellung eines Diphospholans L:

1,2;5,6-Di-*O*-isopropyliden-D-mannitol (1): kommerziell erhältlich bei Firma FLUKA (Best.-Nr. 38410).

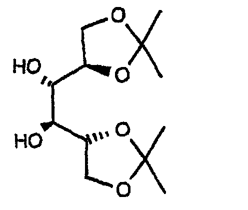

3,4-Di-*O*-benzyl-1,2;5,6-di-*O*-isopropyliden-D-mannitol (2): dargestellt nach: J. Jurcak, T. Bauer, M. Chmielewski, *Carbohydr. Res.* 164 (1987) 493.

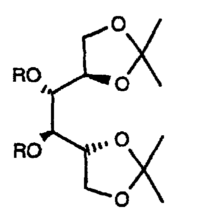

3,4-Di-*O*-benzyl-D-mannitol (3): dargestellt nach: J. Jurcak, T. Bauer, M. Chmielewski, *Carbohydr. Res.* 164 (1987) 493.

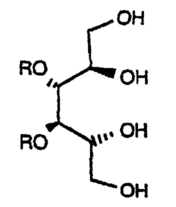

3,4-Di-*O*-benzyl-1,6-di-*O*-toluolsulfonyl-D-mannitol (4): dargestellt nach: J. Fittremann, A. Duréault, J.-C. Depezay, *Tetrahedron Letters* 35 (1994) 1201.

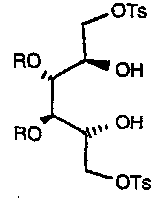

(*2R,3R,4R,5R*)-3,4-Dibenzyloxy-hexan-2,5-diol (5): Eine Lösung bestehend aus 10 g (14.9 mmol) des Ditosylates 4 in 30 ml THF wird bei Raumtemperatur langsam zu einer Suspension von 2.25 g (59.6 mmol) LiAlH₄ in 100 mL THF getropft. Nach einer Stunde Rühren erhitzt man die Suspension zwei Stunden unter Rückfluß. Nach dem Abkühlen zersetzt man das Hydrid durch vorsichtige aufeinanderfolgende Zugabe von 2.25 ml Wasser, 2.25 ml 15 %-iger NaOH und nochmals 6.75 ml Wasser. Die Lösung wird von den ausgefallenen anorganischen Verbindungen abfiltriert und dieser Rückstand mittels Methylenchlorid im Soxhlett extrahiert. Die vereinigten Lösungen werden getrocknet und nach dem Abdestillieren der Lösungsmittel wird der Rückstand säulenchromatographisch gereinigt. (*n*-Hexan:AcOEt = 1:2; R_{f} = 0.45).

Ausbeute: 3.6 g (73 %), weißer Feststoff. Fp.= 46-50°C. [α]²⁶_{D} = -4.7 (*c* 0.990, CHCl₃), ¹H-NMR (CDCl₃): 7.40-7.25 (10 H, m, arom. H), 4.65 (4H, AB-Sp., CH₂Ph, ²J_{A,B}= 11.3 Hz), 4.09 (2H, m, H-2+H-5), 3.53 (2H, m, H-3+H-4), 2.96 (2H, s (Br), 2xOH), 1.25 (6H, d, 2xCH₃, ³J_{H,H}= 6.4Hz); ¹³C-NMR (CDCl₃): 137.4, 128.5, 128.2, 128.0 (arom. C), 81.5 (C-3+C-4), 73.3 (2xCH₂Ph), 67.3 (C-2+C-5), 19.7 (2xCH₃); IR (KBr): 3417, 3287, 3031, 2987, 2965, 2934, 2882, 1455, 1316, 1210, 1112, 1092, 1075, 1056, 1028, 764, 726, 697; MS (70 eV, m/z): 331 [M⁺+H] (1), 297 [M⁺-CH₃-H₂O] (1), 285 [M-C₂H₅O] (2); C₂₀H₂₆O₄ (330.43) ber: C: 72.70 % H: 7.93 %; gef.: C: 72.79 % H: 7.94 %;

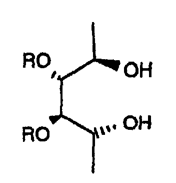

(*4R,5R,6R,7R*)-5,6-Dibenzyloxy-4,7-dimethyl-[1,3,2]dioxathiepan 2,2-dioxid (6): 4.75 g (14.4 mmol) des Diols 5 werden in 20 ml Tetrachlorkohlenstoff mit 1.3 ml Thionylchlorid 1,5 h unter Rückfluß erhitzt. Nach dem Abkühlen entfernt man das Lösungsmittel am Rotationsverdampfer und nimmt den erhaltenen Rückstand mit 10 ml Tetrachlorkohlenstoff, 10 ml Acetonitril und 15 ml Wasser auf. Zu der auf 0°C abgekühlten Lösung gibt man 0.021 g (0.08 mmol) RuCl₃*3H₂O und anschließend 6.2 g (29.0 mmol) Natriumperiodat. Nach einstündigem Rühren bei Raumtemperatur versetzt man die Lösung mit 75 ml Wasser und extrahiert mit 4 x 100 ml Diethylether. Die vereinigten Extrakte werden einmal mit gesättigter NaCl-Lösung gewaschen, anschließend mit Na₂SO₄ getrocknet und durch Kieselgur filtriert. Die etherische Lösung engt man ein und reinigt das cyclische Sulfat 6 durch Säulenchromatographie (n-Hexan : AcOEt = 9 : 1, R_{f} = 0.25).

Ausbeute 3.4 g (60 %) weiße Kristalle. Fp.= 90-94°C. [α]²³_{D} = -2.8 (*c* 1.012, CHCl₃). ¹H-NMR (CDCl₃): 7.40-7.25 (10 H, m, arom. H), 4.79 (4H, AB-Sp., CH₂Ph, ²J_{A,B}= 10.8 Hz), 4.09 (2H, m, H-2+H-5), 3.55 (2H, m, H-3+H-4), 1.53 (6H, d, 2xCH₃, ³J_{H,H}= 6.4Hz); ¹³C-NMR (CDCl₃): 137.1, 128.6, 128.1, 127.7 (arom. C), 84.2 (C-3+C-4), 79.4 (C-2+C-5), 76.2 (2xCH₂Ph), 17.9 (2xCH₃); IR (KBr): 3090, 3062, 3027, 2989, 2939, 2881, 2861, 1498, 1453, 1395, 1380, 1349, 1208, 1103, 1071, 1020, 949, 899, 841, 750, 741, 703, 699, 611; MS (70 eV, m/z): 392 [M⁺] (1), 301 [M⁺-C₇H₇] (47), 195 [M⁺-C₇H₇-C₇H₆O] (36), 91 [C₇H₇⁺] (100); C₂₀H₂₄O₆S (392.47) ber: C: 61.21 % H: 6.16 % S: 8.17 %; gef.: C: 61.20 % H: 6.24 % S: 8.08 %;

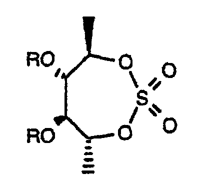

1, 2-Bis((*4S,5S,6S,7S*)-5,6-dibenzyloxy-4,7-dimethyl-phospholanyl)benzen (7): Zu einer Lösung von 0.564 g (3.96 mmol) 1,2-Bis(phosphanyl)benzèn in 70 ml THF gibt man tropfenweise 4.95 ml (7.93 mmol) *n*-BuLi (1.6 M in Hexan) bei Raumtemperatur. Die entstehende gelbe, klare Lösung wird weitere 2 Stunden gerührt und anschließend langsam mit einer Lösung von 3.11 g (7.92 mmol) des cyclischen Sulfats 6 in 15 ml THF versetzt. Dabei erfolgt ein Farbumschlag nach rot-orange. Nach vier Stunden überführt man weitere 5.45 ml (8.71 mmol) *n*-BuLi in das Reaktionsgemisch und läßt weitere 16 h bei Raumtemperatur rühren. Zur Aufarbeitung wird die resultierende rote Lösung mit 3 ml Methanol versetzt und das THF unter Vakuum entfernt. Der Rückstand wird mit 50 ml Methylenchlorid aufgenommen und unter anaeroben Bedingungen mit Wasser (20 ml) gewaschen. Nach dem Trocknen (Na₂SO₄) und Entfernen des Lösungsmittels erfolgt eine chromatographische Reinigung (*n*-Hexan : AcOEt = 9 : 1, R_{f}=0.2). 42 %

Ausbeute farbloser Sirup. ¹H-NMR (C₆D₆): 7.70-7.00 (10 H, m, arom. H), 4.50 (8H, m, 4xCH₂Ph), 4.05-3.93 (4H, m, H-2+H-5), 3.15-2.94 (4H, m, H-2+H-5), 1.47 (6H, m, CH₃), 0.88 (6H, m, CH₃); ¹³C-NMR (C₆D₆): 143.3 (m), 139.3, 139.3, 128.5-127.5 (arom. C), 85.2+84.2 (C-3+C-4), 72.2+72.0 (4xCH₂Ph), 32.4 (m, C-2+C-5), 14.5 (CH₃), 13.4 (CH₃); ³¹P-NMR (C₆D₆): -3.4; MS (FDₚₒₛ): 731 [M⁺+H] (100);

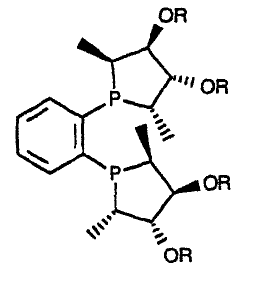

### Beispiel 3

### Katalysatorherstellung:

Cyclooctadienrutheniumbis(2-methallyl) (100 mg, 0.32 mmol) und 0.32 mmol des Phospholanliganden werden in 5 ml Heptan vorgelegt und 12 h bei 60-70°C gerührt. Das Lösungsmittel wird abgezogen, der Rückstand in 5 ml Methylethylketon oder Aceton aufgenommen und mit 2 eq. methanolischer HBr versetzt. Das Gemisch wird 2 h bei RT gerührt, filtriert und eingeengt. Das Produkt ist der Phospholano-ruthenium-dibromkomplex (=Bn-Ro-PHOS).

Statt HBr können auch andere Säuren wie HCl, HI, TFA, HBF₄ und ähnliche verwendet werden. Erhalten werden dann Komplexe mit den entsprechenden Gegenionen.

### Beispiel 4

### Hydrierung:

Der Katalysator aus Beispiel 3 wird in Methanol gelöst und mit 10000 eq. β-Ketoester versetzt. Optional kann noch Wasser und eine Säure (0,5 - 2 eq. (bezogen auf Katalysator) einer anorganischen Mineralsäure oder starken organischen Säure, wie TFA, Trichloressigsäure u.ä.) zugegeben werden. Nach Aufpressen von Wasserstoff ( 10 bar ) wird bei 35 °C gerührt, bis die Wasserstoffaufnahme beendet ist.

### Beispiele:

Folgende Experimente wurden nach o.a. Vorschrift durchgeführt.

| Ligand | Substrat | S : K | MeOH /H₂O | Temp. | Druck H₂ | Zeit | Umsatz | %*ee* |
|---|---|---|---|---|---|---|---|---|
| | | | | | | | | |
| Bn-Ro-PHOS | 3-Oxo-1,8-octansäure-dimethylester | 10000 : 1 | 10 : 1 | 35 | 10 | 72 | 100 | 96.6 (S) |
| Bn-Ro-PHOS | 3-Oxo-1,8-octansäure-dimethylester | 30000 : 1 | 15 : 1 | 35 | 30 | 24 | 97 | 95.8 (S) |
| Bn-Ro-PHOS | 3-Oxo-1,8-octan-säure-dimethylester | 30000 : 1 | 15 : 1 +1.0 eq.TFA | 35 | 30 | 24 | 100 | 98.8 (S) |
| Bn-Ro-PHOS | Acetessigsäuremethylester | 15000 : 1 | 15 : 1 | **25** | 10 | 16 | 100 | 94.3 (S) |
| Bn-Ro-PHOS | 3-Oxo-Valeriansäure-methylester | 30000 : 1 | 15 : 1 | 35 | 30 | 16 | 100 | 97.0 (S) |
| Bn-Ro-PHOS | Acetessigsäureethylester | 30000 : 1 | 15 : 1 | **25** | 30 | 16 | 100 | 94.5 (S) |
| | | | | | | | | |

S: K = Substrat/ Katalysator-Verhältnis (w/w)

Die Umsätze und Enantiomerenüberschüsse wurden per HPLC bzw. GC bestimmt.

Analytische Daten des (3S)-3-Hydroxyoctandisäuredimethylesters

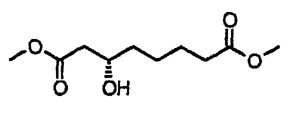

| ¹H-NMR (400 MHz, CDCl₃): | | ¹³C-NMR (100 MHz, CDCl₃): | |
|---|---|---|---|
| δ: | 1.50 (m, 4 H, 5,6-CH₂) 1.67 (m, 2 H, 4-CH₂) 2.35 (t, J= 8 Hz, 7-CH₂) 2.47 (m, 2 H, 2-CH₂) 3.32 (s, 1 H, OH) 3.65 (s, 3 H, OCH₃) 3.70 (s, 3 H, OCH₃) 4.01 (m, 1 H, CH) | δ: | 24.8 (5,6-C) 25.1 (5,6-C) 33.9 (7-C) 36.2 (4-C) 41.4 (2-C) 51.5 (OCH3) 51.7 (OCH3) 67.7 (3-C) 173.2 (Ester-C) 174.1 (Ester-C) |
| b.p.: 165°C/0.2 mbar; [α]_{D}²⁵ : + 14.3 (c= 1.1, CH₂Cl₂) | | | |

3-Hydroxy-pentancarbonsäuremethylester:LIPODEX A, 50 m, 55°C 1H-NMR (CDCl₃): 0.91 (3H, t, CH₃-CH₂, 3J = 7.5 Hz), 1.46 (m, 2H, CH₂-CH₃), 2.36 (1H, dd, Hₐ-CH₂-COOCH₃, 1J = 16.2 Hz, 3J = 8.8 Hz), 2.46 (1H, dd, H_{b}-CH₂-COOCH₃, 1J = 16.2 Hz, 3J = 3.2 Hz), 2.90 (1H, s, OH), 3.66 (3H, s, OCH₃), 3.99 (2H, m, CH-OH);
13C-NMR (CDCl₃): 9.7 (CH₃-CH₂), 29.3 (CH₂-CH₃), 40.7 (CH₂-COOCH₃), 51.6 (OCH₃), 69.2 (CH-OH), 173.4 (COOCH₃);

3-Hydroxy-butancarbonsäuremethylester: LIPODEX A, 50 m, 55°C 1H-NMR (CDCl₃): 1.18 (3H, d, CH₃-CH, 3J = 6.4 Hz), 2.39 (1H, dd, Hₐ-CH₂-COOCH₃, 1J = 16.2 Hz, 3J = 8.3 Hz), 2.44 (1H, dd, H_{b}-CH₂-COOCH₃, 1J = 16.2 Hz, 3J = 3.8 Hz), 2.99 (1H, s, OH), 3.66 (3H, s, OCH₃), 4.15 (2H, m, CH-OH) ;
13C-NMR (CDCl₃): 22.4 (CH₃-CH), 42.5 (CH₂-COOCH₃), 51.6 (OCH₃), 64.1 (CH-OH), 173.2 (COOCH₃) ;

3-Hydroxy-butancarbonsäureethylester: LIPODEX A, 50 m, 50°C 1H-NMR (CDCl₃): 1.18 (3H, d, CH₃-CH, 3J = 6.2 Hz), 1.23 (3H, t, CH₃-CH₂, 3J = 7.2 Hz), 2.39 (1H, dd, Hₐ-CH₂-COOCH₃, 1J = 16.2 Hz, 3*J* = 8.5 Hz), 2.43 (1H, dd, H_{b}-CH₂-COOCH₃, 1J = 16.2 Hz, 3J = 3.8 Hz), 2.91 (1H, s, OH), 4.13 (2H, q, CH₂-CH₃, 3J = 7.2 Hz), 4.16 (2H, m, CH-OH) ;
13C-NMR (CDCl₃): 14.1 (CH₃-CH₂), 22.4 (CH₃-CH), 42.7 (CH₂-COOCH₃), 60.6 (OCH₂), 64.2 (CH-OH), 172.8 (COOCH₃) ;

## Patentansprüche

1. Verfahren zur Herstellung von β-Hydroxyestern, indem man β-Ketoester mit Wasserstoff in Gegenwart von Katalysatoren der Formel LRuX₂ umsetzt, wobei
X Halogen, Acetat, Allyl, Methallyl, 2-Phenylallyl, Perchlorat, Trifluoracetat, Tetrafluoroborat, Hexafluorantimonat, Hexafluorphosphat, Hexafluorarsenat, Trichloracetat
L ein zweizähniges Phospholan der allgemeinen Formel I 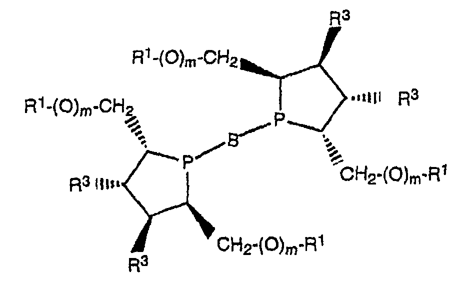
mit
B = ein Brückenglied mit 1 - 5 C-Atomen zwischen den beiden P-Atomen oder 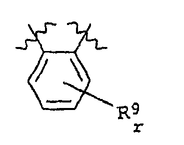
r = 0, 1, 2, 3
R⁹ = Alkyl oder
annelliertes Aryl
R¹ = H, C₁ - C₆-Alkyl, Aryl, Alkylaryl, SiR²₃
R² = Alkyl, Aryl
m = 0,1
R³ = H, OR⁴
R⁴ = R¹
mit der Maßgabe, wenn m = 1, dann R³ = H und wenn m = 0, dann R³ ≠ H, bedeuten.

2. Verfahren nach Anspruch 1, **dadurch gekennzeichnet, daß** die Umsetzung bei einer Temperatur zwischen 0 und 100°C durchgeführt wird.

3. Verfahren nach Anspruch 1, **dadurch gekennzeichnet, daß** die Umsetzung bei einem Wasserstoffdruck von 0 bis 300 bar durchgeführt wird.

4. Verfahren nach Anspruch 1, **dadurch gekennzeichnet, daß** die Umsetzung in einem Alkanol-haltigen Lösungsmittel erfolgt.

5. Verfahren nach Anspruch 4, **dadurch gekennzeichnet, daß** als Lösungsmittel dasjenige Alkanol verwendet wird, aus dem der β-Ketoester aufgebaut ist.

6. Verfahren nach Anspruch 1, **dadurch gekennzeichnet, daß** der Katalysator in einem Gewichtsverhältnis von 1:10 bis 1:1000000, bezogen auf den Ketoester, eingesetzt wird.

7. Verfahren nach Anspruch 1, **dadurch gekennzeichnet, daß** die Umsetzung in Gegenwart einer Säure erfolgt.

8. Verfahren nach Anspruch 1, **dadurch gekennzeichnet, daß** als β-Ketoester eine Verbindung der Formel II 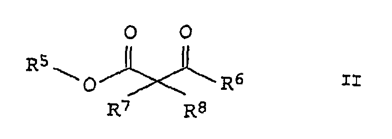 mit
R⁵, R⁶ = Alkyl, Aryl, Alkylaryl, ggf. substituiert
R⁷, R⁸ = H , Alkyl, Aryl, Alkyloxyl, ggf. substituiert,
eingesetzt wird.

9. Verfahren nach Anspruch 8, **dadurch gekennzeichnet, daß**
R⁶ = -CH₂-CH₂-CH₂-CH₂-COOR⁵ und
R⁷, R⁸ = H bedeuten.

## Claims

1. A process for preparing a β-hydroxy ester by reacting a β-keto ester with hydrogen in the presence of a catalyst of the formula LRuX₂ where
X is halogen, acetate, allyl, methallyl, 2-phenylallyl, perchlorate, trifluoroacetate, tetrafluoroborate, hexafluoroantimonate, hexafluorophosphate, hexafluoroarsenate, trichloroacetate,
L is a bidentate phospholane of the formula I 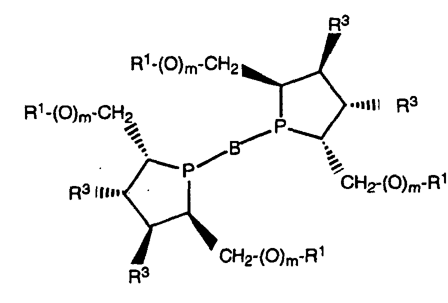
where
B = a bridging link with 1 - 5 carbon atoms between the two phosphorus atoms, or or 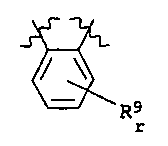
r = 0, 1, 2, 3,
R⁹ = alkyl or fused-on aryl
R¹ = H, C₁ - C₆-alkyl, aryl, alkylaryl or SiR²₃,
R² = alkyl or aryl,
m = 0 or 1,
R³ = H or OR⁴, and
R⁴ = R¹,
with the proviso that if m = 1 then R³ = H and if m = 0 then R³ ≠ H.

2. A process as claimed in claim 1, wherein the reaction is conducted at a temperature between 0 and 100°C.

3. A process as claimed in claim 1, wherein the reaction is conducted at a hydrogen pressure of from 0 to 300 bar.

4. A process as claimed in claim 1, wherein the reaction takes place in a solvent which comprises alkanol.

5. A process as claimed in claim 4, wherein the solvent used is the alkanol on which the β-keto ester is based.

6. A process as claimed in claim 1, wherein the catalyst is employed in a weight ratio of from 1:10 to 1:1,000,000, based on the keto ester.

7. A process as claimed in claim 1, wherein the reaction takes place in the presence of an acid.

8. A process as claimed in claim 1, wherein the β-keto ester employed is a compound of the formula II 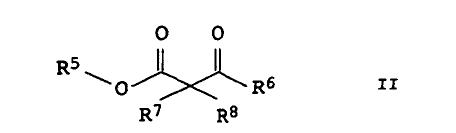 where
R⁵, R⁶ = alkyl, aryl or alkylaryl, substituted or unsubstituted, and
R⁷, R⁸ = H, alkyl, aryl or alkoxy, substituted or unsubstituted.

9. A process as claimed in claim 8, wherein
R⁶ = -CH₂-CH₂-CH₂-CH₂-COOR⁵ and
R⁷ and R⁸ = H.

## Revendications

1. Procédé pour la préparation de β-hydroxyesters, dans lequel on fait réagir un β-cétoester avec de l'hydrogène en présence de catalyseurs de formule LRuX₂, où
X désigne un groupe: halogéno, acétate, allyle, méthallyle, 2-phénylallyle, perchlorate, trifluoroacétate, tétrafluoroborate, hexafluoroantimoniate, hexafluorophosphate, hexafluoroarséniate, trichloroacétate
L représente un phospholane bidenté de formule générale I 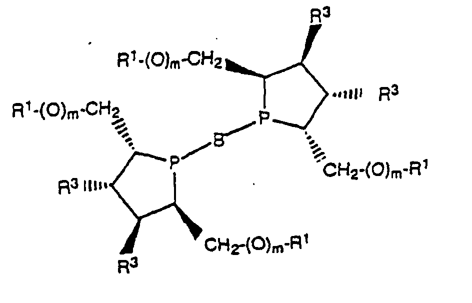
avec
B = un élément pontant ayant 1-5 atomes de carbone entre les deux atomes P ou 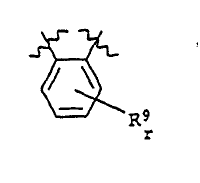
r = 0, 1, 2, 3
R⁹ = alkyle ou aryle condensé
R¹ = H, alkyle en C₁-C₆, aryle, alkylaryle, SiR²₃
R² = alkyle, aryle
m = 0, 1
R³ = H, OR⁴
R⁴ = R¹
avec pour condition que, quand m = 1, alors R³ = H et, quand m = 0, alors R³ ≠ H.

2. Procédé selon la revendication 1, **caractérisé par le fait que** la réaction est mise en oeuvre à une température entre 0 et 100°C.

3. Procédé selon la revendication 1, **caractérisé par le fait que** la réaction est mise en oeuvre sous une pression d'hydrogène de 0 à 300 bar.

4. Procédé selon la revendication 1, **caractérisé par le fait que** la réaction s'effectue dans un solvant contenant un alcanol.

5. Procédé selon la revendication 4, **caractérisé par le fait qu'**on utilise comme solvant l'alcool à partir duquel est élaboré le β-cétoester.

6. Procédé selon la revendication 1, **caractérisé par le fait que** le catalyseur est utilisé dans un rapport en poids de 1:10 à 1:1000000, par rapport au cétoester.

7. Procédé selon la revendication 1, **caractérisé par le fait que** la réaction s'effectue en présence d'un acide.

8. Procédé selon la revendication 1, **caractérisé par le fait qu'**on utilise comme β-cétoester un composé de formule II 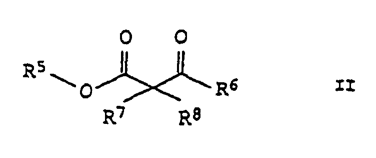 avec
R⁵, R⁶ = alkyle, aryle, alkylaryle, éventuellement substitués
R⁷, R⁸ H, alkyle, aryle, alkyloxyle, éventuellement substitués.

9. Procédé selon la revendication 8, **caractérisé par le fait que**
R⁶ = -CH₂-CH₂-CH₂-CH₂-COOR⁵ et
R⁷, R⁸ = H.
